# EUROPEAN PATENT APPLICATION

(11) **EP 1 818 344 A1**
(43) Date of publication of application: **15.08.2007**
(21) Application number: 05806183.9
(22) Date of filing: 14.11.2005
(51) Int. Cl.: C08B 37/08, C08J 3/24, C08L 5/00

(54) **METHOD FOR PRODUCING CROSSLINKED HYALURONIC ACID GEL**

(30) Priority: 15.11.2004 US 627886 P
(71) Applicant: Shiseido Company, Limited, Chuo-ku Tokyo 104-8010 (JP)
(72) Inventor: YOKOKAWA, Yoshihiro c/o SHISEIDO RESEARCH CENTER, Tsuzuki-ku, Yokohama-shi, Kanagawa 2248558 (JP); OKA, Takashi c/o SHISEIDO RESEARCH CENTER (SHIN, Yokohama-shi, Kanagawa 2248558 (JP); MORI, Yuichiro c/o SHISEIDO RESEARCH CENTER, ku, Yokohama-shi, Kanagawa 2248558 (JP); UENO, Norio c/o SHISEIDO RESEARCH CENTER (SHIN, Yokohama-shi, Kanagawa 2248558 (JP)
(74) Representative: Henkel, Feiler & Hänzel
(86) International application number: PCT/JP2005/020864
(87) International publication number: WO 2006/051950

(57) **Abstract**

A novel process which can simply prepare a crosslinked hyaluronic acid gel having a small crosslinking agent content and exhibiting excellent viscoelasticity is provided.

A process for preparing a crosslinked hyaluronic acid gel, comprising stirring and mixing a mixture containing 10W/V % or more of hyaluronic acid, a crosslinking agent and water under acidic or alkaline condition.

## Description

### RELATED APPLICATIONS

The present application claims the priority of provisional application of Serial Number 60/627,886 filed on November 15, 2004.

### TECHNICAL FIELD

The present invention relates to a process for preparing a crosslinked hyaluronic acid gel, more particularly, a process for more simply preparing a crosslinked hyaluronic acid gel having a low crosslinking rate and excellent viscoelasticity.

### BACKGOUND ART

A crosslinked hyaluronic acid gel obtained by crosslinking hyaluronic acid is excellent in biocompatibility and, at the same time, also has such the biodegradability that it is progressively degraded in a living body with time, and is finally extinguished. Previously, utilizing this nature of a crosslinked hyaluronic acid gel, study and development regarding application to adhesion preventing agents, bone repairing agents, drug sustained release compositions, and tissue increasing substances have been extensively performed. Among them, as a representative example of application to tissue increasing substances, antiwrincle injections in the field of aesthetic plastic surgery.

A crosslinked hyaluronic acid gel is generally prepared by stirring and mixing hyaluronic acid and a crosslinking agent in an aqueous solution, to chemically combine between hyaluronic acid polymer chains by a crosslinking agent. Herein, when such the crosslinked hyaluronic acid gel is administered to a living body, it is feared that a gel is degraded in a living body, a remaining crosslinking agent component is recognized as a foreign matter to a living body, and this adversely influences such as causing an inflammation reaction. For this reason, when maintenance of biocompatibility is intended, it is desired that a crosslinked hyaluronic acid gel is prepared at as a low crosslinking rate as possible.

However, in the previous general crosslinking method, when an amount of a crosslinking agent to be added is reduced, viscoelasticity of the resulting crosslinked hyaluronic acid gel is reduced, and the gel becomes soft and, for example, when it is used as an antiwrinkle injection, a constant volume cannot be maintained at an injected site. In addition, when a crosslinked hyaluronic acid gel is used as a drug sustained release preparation, in order to maintain proper effect of a drug for a constant term, it is required that a drug resides in a living body for a long term, and a denser high viscoelastic crosslinked gel is required. However, when an amount of a crosslinking agent is reduced, it is difficult to obtain such the high viscoelastic crosslinked gel. Like this, it was a very difficult object to prepare a crosslinked hyaluronic acid gel having both of a low crosslinking rate and excellent viscoelasticity.

With respect to this object, Japanese Patent No. 3094074 reports a process for preparing a crosslinked hyaluronic acid gel having a relatively low crosslinking rate and excellent viscoelasticity. However, this process, specifically, is via a two-stage crosslinking reaction step as follows: hyaluronic acid and a crosslinking agent are mixed in an aqueous solution to initiate a crosslinking reaction and, by adding water before occurrence of gelling to dilute a mixed solution, progression of a crosslinking reaction is prevented once, and a crosslinking reaction is progressed again by volatilizing water from this mixed solution. Therefore, there is a problem that operation is very troublesome, control of a reaction is difficult, and the process is not suitable for mass production.
Patent Literature 1 :Japanese Patent No. 3094074

### DISCLOSURE OF THE INVENTION

### PROBLEM TO BE SOLVED BY THE INVENTION

The present invention was done in view of the aforementioned problem of the prior art, and an object thereof is to provide a novel process for simply preparing a crosslinked hyaluronic acid gel having a low crosslinking rate and exhibiting excellent viscoelasticity.

### MEANS OF SOLVING THE PROBLEM

In order to attain the aforementioned object, the present inventors intensively studied and, as a result, it was made clear that, by adopting condition of a high concentration of 10W/V% or higher of hyaluronic acid in a mixture to be subjected to a crosslinking reaction, a crosslinked hyaluronic acid gel having excellent viscoelasticity is obtained even when an amount of a crosslinking agent to be added is decreased. When general hyaluronic acid is used, a hyaluronic acid aqueous solution having a high concentration such as 10W/V% or higher exhibits the solid powder state or the very highly viscous gel state, which is hardly called solution state. That is, the present inventors found out that, by reacting hyaluronic acid under the extremely high concentration condition which is not usually used by a person skilled in the art, a crosslinked hyaluronic acid gel exhibiting excellent viscoelasticity can be easily prepared although a crosslinking rate is low, which resulted in completion of the present invention.

The process for preparing a crosslinked hyaluronic acid gel of the present invention is characterized in that a mixture containing 10W/V% or more of hyaluronic acid, a crosslinking agent and water is stirred and mixed under acidic or alkaline condition. In addition, in the process for preparing a crosslinked hyaluronic acid gel, it is suitable that a storage modulus G' (frequency 1Hz) of the mixture before subjected to a crosslinking reaction is 15000Pa or higher.

In addition, in the process for preparing a crosslinked hyaluronic acid gel, it is preferable that the mixture is stirred and mixed without physical cutting of a hyaluronic acid polymer chain in the mixture. In addition, in the process for preparing a crosslinked hyaluronic acid gel, it is preferable that the mixture is stirred and mixed with a rotation/revolution mixer. In addition, in the process for preparing a crosslinked hyaluronic acid gel, it is preferable that the mixture is stirred and mixed with a dough kneading machine or a rice-cake making machine. In addition, in the process for preparing a crosslinked hyaluronic acid gel, it is preferable that the mixture is stirred and mixed by kneading with a human hand. In addition, in the process for preparing a crosslinked hyaluronic acid gel, it is preferable that a concentration of a crosslinking agent in the mixture is 0.02 to 1 W/V%. In addition, in the process for preparing a crosslinked hyaluronic acid gel, it is preferable that a concentration of a crosslinking agent in the mixture is 0.02 to 2W/W% relative to hyaluronic acid disaccharide repeating unit. In addition, in the process for preparing a crosslinked hyaluronic acid gel, it is preferable that a crosslinking agent is selected from the group consisting of divinylsulfone, 1,4-butanediol diglycidyl ether, and ethylene glycol diglycidyl ether.

### BRIEF DESCRIPTION OF THE DRAWINGS

Fig. 1 is a view of an entire rotation/revolution mixer used in one example of the present invention.
Fig. 2 is a view of an entire dough kneading machine used in one example of the present invention.
Fig. 3 shows results of measurement of viscoelasticity of a crosslinked hyaluronic acid gel (hyaluronic acid 41W/V%, divinylsulfone crosslinking rate 0.8% relative to hyaluronic acid disaccharide repeating unit) obtained by Example 1-1 of the present invention.
Fig. 4 shows results of measurement of viscoelasticity of a commercially available crosslinked hyaluronic acid gel (Restylane: manufactured by Q-MED).
Fig. 5 shows results of measurement of viscoelasticity of a crosslinked hyaluronic acid gel (hyaluronic acid 33W/V%, 1,4-butanediol diglycidyl ether crosslinking rate 1% relative to hyaluronic acid disaccharide repeating unit) obtained in Example 1-2 of the present invention.
Fig. 6 shows results of measurement of viscoelasticity of a crosslinked hyaluronic acid gel (hyaluronic acid 26W/V%, divinylsulfone crosslinking rate 0.8% relative to hyaluronic acid disaccharide repeating unit) obtained in Example 1-3 of the present invention.
Fig. 7 shows results of measurement of viscoelasticity of a crosslinked hyaluronic acid gel (hyaluronic acid 26W/V%, 1,4-butanediol diglycidyl ether crosslinking rate 1% relative to hyaluronic acid disaccharide repeating unit) obtained in Example 1-4 of the present invention.
Fig. 8 shows results of measurement of viscoelasticity of a crosslinked hyaluronic acid gel (hyaluronic acid 13W/V%, 1,4-butanediol diglycidyl ether crosslinking rate 2.8% relative to hyaluronic acid disaccharide repeating unit) obtained in Example 1-5 of the present invention.
Fig. 9 is a view summarizing sample weights after a enzymatic reaction of a test product of a crosslinked hyaluronic acid gel slurry obtained by the process of the present invention and a 1.2% hyaluronic acid aqueous solution in which an enzyme solution is added (and the control), as a theoretical remaining sample percentage (%).

### DESCRIPTION OF THE PREFERRED EMBODIMENT

Embodiments of the present invention will be explained in detail below, but the present invention is not limited by them.
The process for preparing a crosslinked hyaluronic acid gel of the present invention is characterized in that a mixture containing 10W/V% or more of hyaluronic acid, a crosslinking agent and water is stirred and mixed under acidic or alkaline condition. Thereby, a crosslinked hyaluronic acid gel having excellent viscoelasticity can be simply prepared although the crosslinking rate is low.

In the present invention, "crosslinking rate" represents that the number of crosslinking agents per hyaluronic acid disaccharide repeating unit. For example, 100% crosslinking rate indicates one crosslinking agent per one hyaluronic acid disaccharide repeating unit.
Hyaluronic acid used in the present invention is a straight-chain polymer in which a N-acetyl-D-glucosamine residue and a D-glucuronic acid residue are bound alternately as shown by the following general formula, and as far as hyaluronic acid has such a composition, it can be used without any limitation.

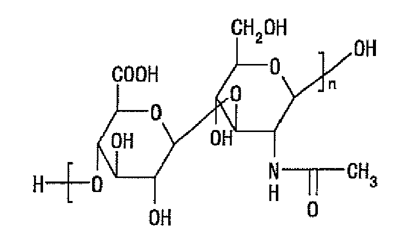

Hyaluronic acid can be obtained, for example, by isolation and extraction from a chicken crest or other animal tissue, or a fermentation method using a microorganism such as genus Streptococcus. In addition, in the present invention, for example, as a derivative of hyaluronic acid, a hyaluronic acid metal salt such as a sodium hyaluronate salt, a potassium hyaluronate salt and the like, or a hyaluronic acid derivative obtained by etherifying, esterifying, amidating, acetalizing, or ketalizing a hydroxyl group, a carboxyl group or the like of hyaluronic acid can be used.

Alternatively, as hyaluronic acid in the present invention, a commercially available product may be used. Examples of commercially available hyaluronic acid include Biohyalo 12 (manufactured by Shiseido), hyaluronic acid (manufactured by Kibun) and the like.

In the present invention, it is necessary that a mixture to be subjected to a crosslinking reaction contains the hyaluronic acid at 1 0W/V% or more. In a mixture containing hyaluronic acid at a high concentration like this, since hyaluronic acid molecular chains are present in the state where they are entangled very complicatedly, molecular chains sterically restrain each other by partial crosslinking of hyaluronic acid chains, and a network structure can be stabilized firm. And, for this reason, it is considered that, by using a mixture containing 10W/V% or more of hyaluronic acid in a crosslinking reaction, a crosslinked hyaluronic acid gel exhibiting excellent viscoelasticity can be prepared although a crosslinking rate is low. On the other hand, in the case where a hyaluronic acid concentration is lower than 10W/V%, when a crosslinking rate is low, a freedom degree of a hyaluronic acid molecular chain is high and, as a result, a crosslinked gel becomes soft, and excellent viscoelasticity is not obtained. In addition, in the present invention, it is preferable that a mixture to be subjected to a crosslinking reaction contains 10 to 20W/V% of the hyaluronic acid.

Usually, when generally used hyaluronic acid having a molecular weight of 100 thousands or higher is used to prepare a hyaluronic acid aqueous solution having a high concentration of the aforementioned 10W/V% or more, the solid powder state or the very highly viscous gel state is exhibited, and a storage modulus G' at a frequency of 1Hz is 15000Pa or higher. In the present invention, it is preferable that a reaction is performed in the state where a mixture to be subjected to a crosslinking reaction exhibits the solid powder state or the highly viscous gel state. That is, in the present invention, it is necessary that a crosslinking reaction is performed in the state where the hyaluronic acid molecules are entangled very complicatedly and, even if a hyaluronic acid concentration is 10W/V% or higher, when a reaction is performed in the solution state where hyaluronic acid is dispersed in water, a crosslinked hyaluronic acid gel having desired viscoelasticity is not obtained in some cases. For this reason, in the present invention, it is preferable that a storage modulus G' (frequency 1Hz) of a mixture to be subjected to a crosslinking reaction is 15000Pa or higher.

A molecular weight of hyaluronic acid used in the present invention is not particularly limited, but a molecular weight of 100 thousands or higher, further around 500 thousands to 3 million is preferable. Usually, generally used hyaluronic acid has a molecular weight of 100 thousands or higher in almost cases, but hyaluronic acid having a molecular weight of around 10 thousands, a molecular weight of which has been specially reduced, is also present. In the present invention, when this low-molecular hyaluronic acid having a molecular weight of around 10 thousands is used, even if a hyaluronic acid concentration is 10W/V% or higher, hyaluronic acid is uniformly dispersed in water into the solution state in some cases and, when a crosslinking rate is low, a gel becomes soft, and desired viscoelasticity is not obtained in some cases, being not preferable.

As a crosslinking agent used in the present invention, any crosslinking agent may be used as far as it can crosslink between polymer chains of the aforementioned hyaluronic acid by a chemical bond. As a crosslinking agent for hyaluronic acid, a polyfunctional compound having two or more functional groups which can react with a reactive functional group in a hyaluronic acid molecule, such as a carboxyl group, a hydroxyl group and an acetamide group, to form a covalent bond can be used. Examples of a crosslinking agent used in the present invention include alkyldiepoxy bodies such as 1,3-butadiene diepoxide, 1,2,7,8-diepoxyoctane, 1,5-hexadiene diepoxide and the like, diglycidyl ether bodies such as ethylene glycol diglycidyl ether, 1,4-butanediol diglycidyl ether, bisphenol A diglycidyl ether and the like, divinylsulfone, epichlorohydrin. Among them, particularly, divinylsulfone, 1,4-butanediol diglycidyl ether, and ethylene glycol diglycidyl ether can be suitably used. In the present invention, two or more kinds of crosslinking agents may be used by appropriately combining them.

In the present invention, an amount of the crosslinking agent to be blended in a mixture which is subjected to a crosslinking reaction is not particularly limited, but from a viewpoint of biocompatibility of the resulting crosslinked hyaluronic acid gel, it is preferable to perform a crosslinking reaction with an as small amount as possible of a crosslinking agent. Specifically, a concentration of the crosslinking agent in a mixture to be subjected to a crosslinking reaction is preferably 0.02 to 1W/V%, more preferably 0.05 to 0.5W/V%. Alternatively, it is preferable that a concentration of a crosslinking agent in a mixture is 0.02 to 2W/W% relative to hyaluronic acid disaccharide repeating unit. According to the present invention, even when a crosslinking agent concentration in a mixture is 1W/V% or lower, a crosslinked hyaluronic acid gel having excellent viscoelasticity can be prepared. In the present invention, when a general low-molecular crosslinking agent is used, and a crosslinking reaction is performed using a crosslinking agent concentration of 0.066W/V%, and a hyaluronic acid concentration of 14W/V%, under presumption that a total amount of a crosslinking agent is reacted with a reactive group of hyaluronic acid, a crosslinking rate of the resulting crosslinked hyaluronic acid gel is usually about 1.67% or lower per one unit of a hyaluronic acid disaccharide repeating unit. Put another way, 1.67% crosslinking rate indicates one crosslinking agent per 64 hyaluronic acid disaccharide repeating unit.

In the process for preparing a crosslinked hyaluronic acid gel of the present invention, by stirring and mixing a mixture containing 10W/V% or more of the hyaluronic acid, the crosslinking agent and water under acidic or alkaline condition, to react a reactive functional group of the hyaluronic acid and the crosslinking agent, hyaluronic acid polymer chains are crosslinked by a chemical bond to produce a crosslinked hyaluronic acid gel.

In the present invention, for the purpose of enhancing reactivity of hyaluronic acid at a crosslinking reaction, a pH of a mixture is appropriately adjusted with an acid such as hydrochloric acid, sulfuric acid and the like, a base such as sodium hydroxide, potassium hydroxide and the like, or a suitable buffer such as a phosphate salt, a quaternary ammonium salt or the like, and stirring and mixing are performed under acidic or alkaline condition. Specifically, for example, it is preferable that a pH of a mixture is adjusted to 1 to 5 under acidic condition, or 10 to 14 under alkaline condition.

In addition, in the present invention, in addition to the aforementioned essential components, components which are usually used in medicaments, cosmetics or the like may be blended in a mixture to be subjected to a crosslinking reaction in advance, in such a range that the object and the effect of the present invention are not influenced. Examples of components which can be blended include ascorbic acid and a derivative thereof, a humectant such as glycerin and the like, retinol and a derivative thereof, and an anti-inflammatory agent such as salicylic acid and the like.

In the present invention, the method of stirring and mixing a mixture is not particularly limited, but it is preferable to perform stirring and mixing without physical cutting of a hyaluronic acid polymer chain in a mixture. Hyaluronic acid as extremely high water swelling property and, for example, an about 10W/V% hyaluronic acid aqueous solution exhibits the extremely highly viscous gel state. For this reason, for example, in the case of a rotation-type stirring and mixing apparatus such as a propeller mixer and a disper which are generally widely used, a gel is adhered to a stirring wing or an apparatus wall, and it is difficult to uniformly stir and mix an entire system. In addition, when using such a stirring and mixing apparatus, a stirring wing is rotated forcibly to perform stirring and mixing, a molecular chain of a hyaluronic acid polymer is cut with a sharp stirring wing, and an objective crosslinked hyaluronic acid gel (a crosslinked gel having a three-dimensional network structure) is not obtained. Like this, since it was very difficult to perform a uniform crosslinking reaction in a hyaluronic acid aqueous solution under the high concentration condition using a normal stirring and mixing apparatus, previously, upon preparation of a crosslinked hyaluronic acid gel, a crosslinking reaction was generally performed in an around 0.1 to 3W/V% hyaluronic acid aqueous solution having a low viscosity.

To the contrary, in the process for preparing a crosslinked hyaluronic acid gel of the present invention, by performing stirring and mixing without physical cutting of a hyaluronic acid polymer chain in a mixture such as stirring and mixing using a rotation/revolution mixer, stirring and mixing using a dough kneading machine (or rice-cake making machine), and stirring and mixing by kneading with a human hand, it becomes possible to easily form a uniform crosslinked structure even under the extremely high hyaluronic acid concentration condition such as 10 W/V% or higher. That is, according to these stirring and mixing methods, even under the hyaluronic acid high concentration condition, an entire system can be uniformly stirred and mixed without physical cutting of a molecular chain of a hyaluronic acid polymer, and it becomes possible to prepare a uniformly crosslinked hyaluronic acid gel.

The process for preparing a crosslinked hyaluronic acid gel of the present invention will be explained in more detail below by way of embodiments of a stirring and mixing method.

### 1. Stirring and mixing by rotation/revolution mixer

As the method of stirring and mixing a mixture in the present invention, stirring and mixing with a rotation/revolution mixer can be used.

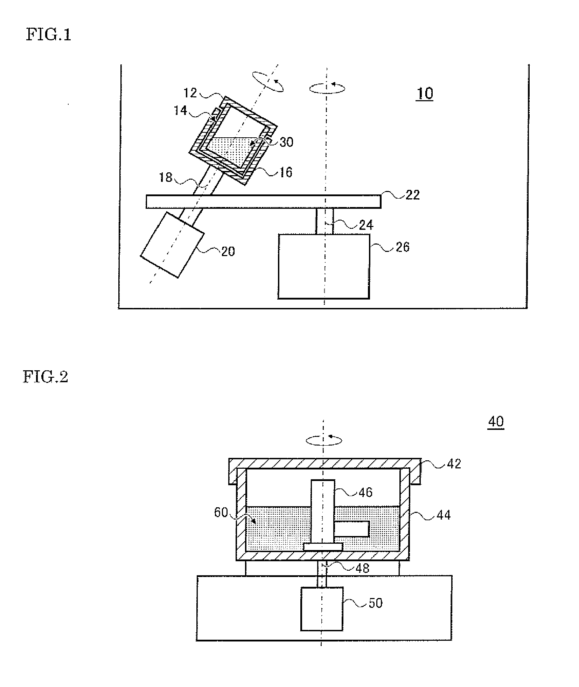

The rotation/revolution mixer used in the present invention is known to a person skilled in the art and, for example, mixing apparatuses described in JP-A No.61-290946, JP-B No.5-32110, JP-A No.10-43568, JP-A No.11-226376, and JP-A No.2000-271465 can be used in the present invention.
A rotation/revolution mixer relating to one example of the present invention is shown in Fig. 1.
A rotation/revolution mixer 10 relating to one example of the present invention is provided with a container body 14 for accommodating a sample, and a lid 12 of the container, a container holder 16 for securing and retaining the container body 14, a rotation mechanism 20 which rotates the container holder 16 along a rotation axis 18, a supporting part 22 for supporting the rotation mechanism 20, and a revolution mechanism 26 for rotating the supporting part 22 along a revolution axis 24.

### (Preparation Example 1)

1) A mixture 30 containing 10W/V% or more of hyaluronic acid, a crosslinking agent and water is accommodated in a container body 14 of a rotation/revolution mixer 10, a container lid 12 is fitted, and this is mounted on a container holder 16.
2) The rotation/revolution mixer 10 is operated to rotate a rotation mechanism 20 and a revolution mechanism 26.
The mixture 30 contains hyaluronic acid at an extremely high concentration of I 0W/V% or higher, and exhibits the solid powder state or the very highly viscous gel state.

In the aforementioned Preparation Example, the container holder 16 is rotated by the rotation mechanism 20 along a rotation axis 18 and, further, a supporting part 22 supporting the rotation mechanism 20 is rotated by the revolution mechanism 26 along a revolution axis 24. And, the mixture 30 accommodated in the container holder 16 is rotated along the rotation axis 18 and, at the same time, is revolved along the revolution axis 24.
As described above, the mixture 30 containing 1 0W/V% or more of hyaluronic acid, a crosslinking agent and water is subjected to the rotation/revolution mixer 10 having the rotation mechanism 20 and the revolution mechanism 26, and stirring and mixing are performed by revolution while rotated. Thereby, although the mixture 30 is in the solid powder state or the very highly viscous gel state, since an entire system is uniformly stirred and mixed without physical cutting of a molecular chain of a hyaluronic acid polymer, it becomes possible to easily form a uniform crosslinking structure between hyaluronic acid molecules.

In addition, the container holder 16 can perform rotation and revolution simultaneously by the rotation mechanism 20 and the revolution mechanism 26, and a rotation /revolution rate can be freely set. A rotation/revolution rate of the container holder 16 is different depending on a nature and a volume of a prepared crosslinked hyaluronic acid gel, and a scale of the container holder 16 and the rotation/revolution mixer 10, and usually, a rotation rate is around 60 to 1000 rpm, and a revolution rate is around 300 to 3000 rpm. In addition, a time for stirring and mixing the mixture 30 with the rotation/revolution mixer 10 is different depending on the various conditions such as a nature and a volume of a prepared crosslinked hyaluronic acid gel and a scale of an apparatus as in the aforementioned rotation rate, but is usually around 10 seconds to 30 minutes.

### 2. Stirring and mixing with dough kneading machine

As the method of stirring and mixing a mixture in the present invention, stirring and mixing with a dough kneading machine can be used.
A dough kneading machine used in the present invention is known to a person skilled in the art, and various commercially available machines can be used in the present invention.
Fig. 2 shows a dough kneading machine relating to one example of the present invention.

A dough kneading machine 40 relating to one example of the present invention is provided with a container body 44 for accommodating a sample, a lid 42 of the container, a dough kneading wing 46 in the container body 44, and a rotation mechanism 50 which rotates the container body 44 along a rotation axis 48. Herein, the dough kneading wing 46 is designed depending on the purpose of kneading dough, unlike a sharp stirring wing for a usual mixer or food processor. Usual dough forms a crosslinked structure of gluten by kneading, and affords peculiar viscoelasticity. And, almost similarly as in the present invention, it is necessary that stirring and mixing are performed without physical cutting of a molecular chain of gluten (this is also true in the case of rice-cake making for obtaining viscoelasticity by entanglement of amylopectin).

### (Preparation Example 2)

1) A dough kneading wing 46 is mounted on a container body 44 of a dough kneading machine 40, a mixture 60 containing 10W/V% or more of hyaluronic acid, a crosslinking agent and water is accommodated in this container body 14, and a container lid 42 is fitted.
2) The dough kneading machine 40 is operated to rotate a rotation mechanism 50.
In the aforementioned Preparation Example, the dough kneading wing 46 in the container body 44 is rotated by the rotation mechanism 50 along a rotation axis 48. And, by rotation of the dough kneading wing 46, the mixture 60 accommodated in the container body 44 is stirred and mixed. Herein, the dough kneading wing 46 is originally designed so that stirring and mixing can be performed without physical cutting of a molecular chain of gluten in dough. For this reason, also in the aforementioned Preparation Example, an entire system is uniformly stirred and mixed without physical cutting of a molecular chain of a hyaluronic acid polymer in the mixture 60.

As described above, by subjecting the mixture 60 containing 10W/V% or more of hyaluronic acid, a crosslinking agent and water to the dough kneading machine 40, and performing stirring and mixing by rotation of the dough kneading wing 46, although the mixture 60 is in the solid powder state or the very highly viscous gel state, an entire system is uniformly stirred and mixed without physical cutting of a molecular chain of a hyaluronic acid polymer. Therefore, it becomes possible to easily form a uniform crosslinked structure between hyaluronic acid molecules.

A rotation rate of the dough kneading wing 46 can be freely set, and is different depending on a nature and a volume of a prepared crosslinked hyaluronic acid gel, a shape of the dough kneading wing 46 and, further, a scale of the container body 44 and the dough kneading machine 40 and, usually, a rotation rate is around 1000 to 2000 rpm. In addition, a time for stirring and mixing the mixture 60 with the dough kneading machine 40 is different depending on various conditions such as a nature and a volume of a crosslinked hyaluronic acid gel, a shape of a wing and a scale of an apparatus as in the case of the aforementioned rotation rate and, usually, the time is around 2 to 6 minutes.

In addition, as the method of stirring and mixing a mixture in the present invention, stirring and mixing with a rice-cake making machine can be used according to the same manner as that of the dough kneading machine.
A rice-cake making machine used in the present invention is known to a person skilled in the art, and various commercially available apparatuses can be used in the present invention.

### 3. Stirring and mixing by kneading with human hand

As the method of stirring and mixing a mixture in the present invention, stirring and mixing by kneading with a human hand can be used.

### (Preparation Example 3)

1) A mixture containing 10W/V% or more of hyaluronic acid, a crosslinking agent and water is placed into a sealable plastic bag, and the bag is sealed.
2) A mixture in the bag is mixed well by kneading with two fingers of both hands.
As described above, regarding a mixture containing 10W/V% or more of hyaluronic acid, a crosslinking agent and water, stirring and mixing are performed by kneading with a human hand, although the mixture is in the solid powder state or the very highly viscous gel state, an entire system is uniformly stirred and mixed without physical cutting of a molecular chain of a hyaluronic acid polymer. Therefore, it becomes possible to form a uniform crosslinked structure between hyaluronic acid molecules.

In addition, a time for mixing the mixture by kneading with a human hand is different depending on various conditions such as a nature and a volume of a prepared crosslinked hyaluronic acid gel, and a size of a bag and, usually, the time is around 5 to 10 minutes.
According to the process for preparing a crosslinked hyaluronic acid gel relating to the present invention explained above, specifically, although a crosslinking rate is a low crosslinking rate of 2% or lower per one unit of a hyaluronic acid disaccharide repeating unit, for example, it becomes possible to simply prepare a crosslinked hyaluronic acid gel having very excellent viscoelasticity in which a storage modulus G' at a frequency of 1Hz of the crosslinked hyaluronic acid gel which has reached swelling equilibrium in a physiological saline is 100Pa or higher, more preferably 1000Pa or higher.

A crosslinked hyaluronic acid gel obtained by the process of the present invention, alone or by blending in a suitable formulation, can be used in medicaments, cosmetics or the like. In addition, according to the process of the present invention, since a crosslinked hyaluronic acid gel exhibiting excellent viscoelasticity can be simply prepared although a crosslinking rate is low, the gel can be applied to substances which are applied in a living body, for example, can be suitably applied to tissue increasing substances such as antiwrincle injections, drug sustained release compositions, adhesion preventing agents, or bone repairing agents.

In addition, since the crosslinked hyaluronic acid gel obtained by the process of the present invention has high enzyme digestion resistance (hyaluronidase resistance), and can maintain a gel structure in a living body for a long term, it is expected that the gel can be particularly preferably applied, for example, as joint injectables, intraocular vitreous body replacing agents, or ophthalmologic operation adjuvant.

Currently, for the purpose of improving symptom of knee arthralgia in knee osteoarthritis, shoulder periarthritis, and rheumatoid arthritis, hyaluronic acid is used as a joint injectable. Since a crosslinked hyaluronic acid gel obtained by the present invention has high enzyme digestion resistance, by using the gel as a joint injectable, it can be expected that the effect of improving symptom can be continued for a longer term than the previous product. In addition, as an intraocular vitreous body substitute for recovering a peeled retina, currently, silicone oil is used widely. Although it is thought that hyaluronic acid which is a component in a living body is suitable as such the intraocular vitreous body substitute, since hyaluronic acid is easily degraded by an enzyme in eyes, currently, hyaluronic acid as a vitreous body substitute is not commercially available. Since a crosslinked hyaluronic acid gel obtained by the present invention has high enzyme digestion resistance, by using the gel as a vitreous body substitute, the effect of residing in eyes over a long period of time, adhering a retina to choroidea, and recovering a retina can be expected. In addition, recently, by using hyaluronic acid as an ophthalmologic operation adjuvant, operation of cataract has been performed safely and easily. And, a crosslinked hyaluronic acid gel obtained by the present invention, although it is a crosslinked gel, is very small fine particles, and exhibits viscoelasticity equivalent to that of a highly viscoelastic hyaluronic acid solution. For this reason, like a commercially available hyaluronic acid ophthalmologic operation adjuvant, it can be expected that the gel is used as an adjuvant at ophthalmologic operation. Further, although commercially available hyaluronic acid preparations are all filtration-sterilized, since the crosslinked hyaluronic acid gel obtained by the present invention can be sterilized with high pressure steam, a risk of pollution of a final product with a microorganism can be minimized.

The present invention will be explained in more detail below by way of Examples of the present invention, but the present invention is not limited to them.

### Preparation of crosslinked hyaluronic acid gel under hyaluronic acid high concentration (rotation/revolution mixer)

The present inventors first tried a crosslinking reaction with a small amount of a crosslinking agent under extremely high hyaluronic acid concentration condition such as 41W/V% with a rotation/revolution mixer, viscoelasticity of the resulting crosslinked hyaluronic acid gel was measured, and this was compared with viscoelasticity of a commercially available crosslinked hyaluronic acid gel. In this application, the volume of hyaluronic acid solution was measured, and W/V% was caluculated.

### Example 1-1: Hyaluronic acid 41W/V%, divinylsulfone crosslinking rate 0.8%

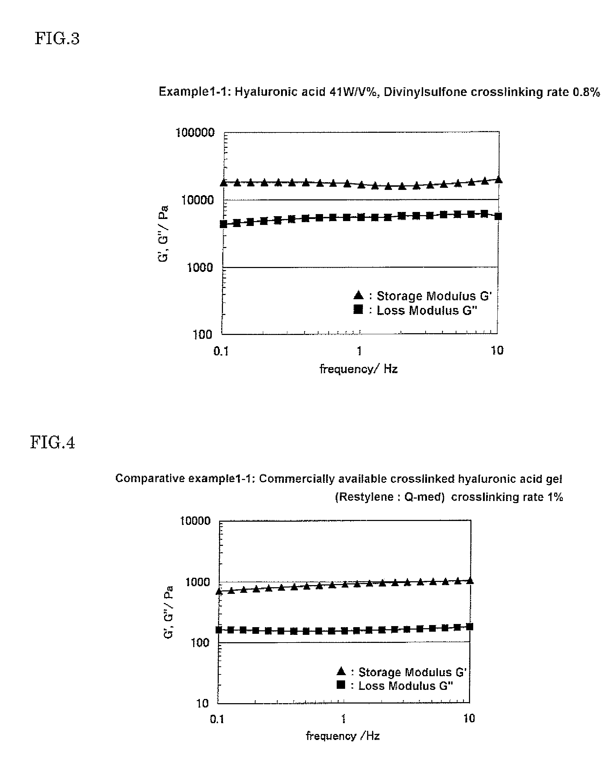

200µL of a 2N NaOH aqueous solution, and 2µL of divinylsulfone were added to 1800µL of purified water and, further, 1.0g of hyaluronic acid (Biohyalo 12: manufactured by Shiseido) was added. This mixture (storage modulus G' at frequency 1Hz: 300000Pa) was stirred and mixed at room temperature for 5 minutes with a rotation/revolution mixer (AR-250: manufactured by THINKY). The product was allowed to stand in a physiological saline for one week until it reached swelling equilibrium. The swollen crosslinking hyaluronic acid gel was crushed with a sample mill (SK-M2: manufactured by KYORITSU RIKO), to obtain a desired crosslinked hyaluronic acid gel slurry. A storage modulus G' and a loss modulus G" of the resulting crosslinked hyaluronic acid gel were measured using a rheometer (Rheolyst AR 1000-N: manufactured by TA Instruments) under the condition of 25°C and a frequency of 0.1 to 10Hz. Under the presumption that a total amount of an added crosslinking agent reacted with a reactive group of hyaluronic acid, a crosslinking rate of the resulting crosslinked hyaluronic acid gel is 0.8% per one unit of a hyaluronic acid disaccharide repeating unit. The results of measurement are shown in Fig. 3.

### Comparative Example 1-1: commercially available crosslinked hyaluronic acid gel (Restylane: manufactured by Q-MED) crosslinking rate 1 %

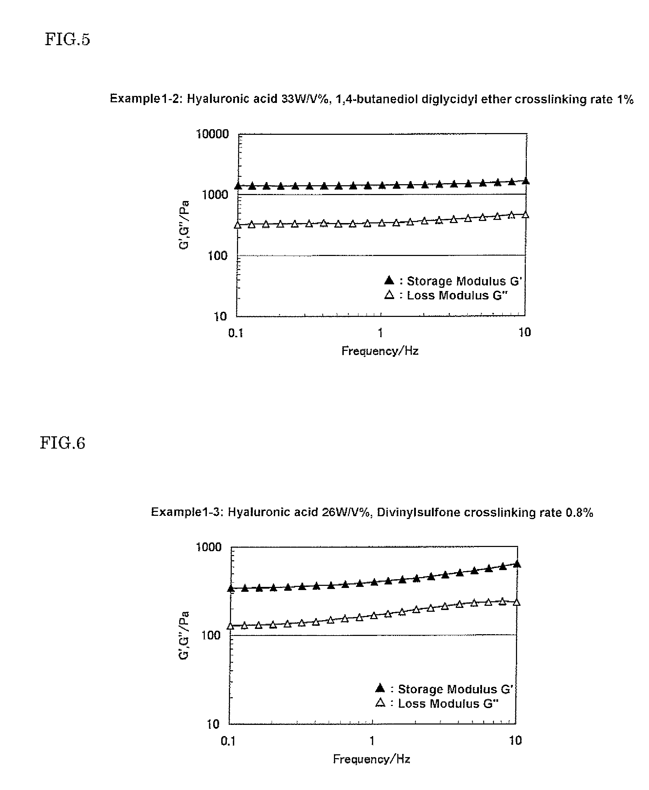

For the purpose of comparison with the present invention, regarding a commercially available crosslinked hyaluronic acid gel (Restylane: manufactured by Q-MED) which has previously been used widely as an antiwrinkle injection, a storage modulus G' and a loss modulus G" were measured as in Example 1-1. This commercially available crosslinked hyaluronic acid gel was prepared by the method described in Japanese Patent No.3094074, and it is presumed that a crosslinking rate is 1% per one unit of a hyaluronic acid disaccharide repeating unit. The results of measurement are shown in Fig. 4.

As shown in Fig. 3, it was revealed that a crosslinked hyaluronic acid gel slurry obtained in Example 1-1, although a crosslinking rate is low as 0.8%, exhibits excellent viscoelasticity of a storage modulus G' of about 20000Pa and a loss modulus G" of about 5000Pa in a range of a frequency 0.1 to 10Hz when the gel reaches swelling equilibrium with a physiological saline. This is thought as follows: Since a hyaluronic acid concentration in a mixture to be subjected to a crosslinking reaction is remarkably high as 41 W/V%, only by crosslinking with a small amount of a crosslinking agent, hyaluronic acid molecular chains are sterically restrained, and a network structure is stabilized firm. On the other hand, as shown in Fig. 4, it was seen that, in a commercially available hyaluronic acid gel slurry of Comparative Example 1-1 which has previously been used widely as an antiwrinkle injection, a crosslinking rate is around 1%, and a storage modulus G' is about 1000Pa, and a loss modulus G" is about 200Pa when the gel reaches swelling equilibrium with a physiological serine. From this, it is seen that the crosslinked hyaluronic acid gel slurry obtained in Example 1-1 has excellent viscoelasticity to a sufficient degree of actual use as an antiwrinkle injection although a crosslinking rate is low.

In addition, although the commercially available crosslinked hyaluronic acid gel slurry used as Comparative Example 1-1 has a low crosslinking rate, a process for preparing it is, as described in Japanese Patent No.3094074, via a two-stage crosslinking reaction step of mixing hyaluronic acid and a crosslinked agent in an aqueous solution to initiate a crosslinking reaction, adding water before occurrence of gelling to dilute a mixed solution, thereby, preventing progression of a crosslinking reaction once, and volatilizing water from this mixed solution to progress a crosslinking reaction again, and very complicated procedure is required. It is seen that, to the contrary, according to the process of Example 1-1, by performing a crosslinking reaction under the extremely high hyaluronic acid concentration condition of 41W/V%, a crosslinked hyaluronic acid gel having a low crosslinking rate and exhibiting excellent viscoelasticity can be prepared more simply.
Subsequently, the present inventors tried the same test as that of the Example 1-1 by appropriately changing a kind of a crosslinking agent and a hyaluronic acid concentration condition to be subjected to a crosslinking reaction, and studied relationship with viscoelasticity of the resulting crosslinked hyaluronic acid gel slurry.

### Example 1-2: 33W/V% hyaluronic acid. 1,4-butanediol diglycidyl ether crosslinking rate 1%

To 2250 µL of purified water were added 250µL of a 2N NaOH aqueous solution, and 5µL of 1,4-butanediol glycidyl ether and, further, 1.0g of hyaluronic acid (Biohyalo 12: manufactured by Shiseido) was added. This mixture (storage modulus G' at frequency of 1Hz: 100000Pa) was stirred and mixed with a rotation/revolution mixer (AR250: manufactured by THINKY) at room temperature for 5 minutes, and this was allowed to stand at room temperature for 24 hours. The resulting gel was allowed to stand in a physiological saline for one week until it reached swelling equilibrium. The swollen crosslinking hyaluronic acid gel was crushed with a sample mill (SK-M2: manufactured by KYORITSU RIKO), to obtain a desired crosslinked hyaluronic acid gel slurry. The resulting crosslinked hyaluronic acid gel slurry was measured for a storage modulus G' and a loss modulus G" as in Example 1-1. Under the presumption that a total amount of the added crosslinking agent reacted with a reactive group of hyaluronic acid, a crosslinking rate of the resulting crosslinked hyaluronic acid gel is 1% per one unit of a hyaluronic acid disaccharide repeating unit. The results of measurement are shown in Fig.5.

As shown in Fig.5, although the crosslinked hyaluronic acid gel of Example 1-2 obtained under the condition of hyaluronic acid concentration 33W/V% using 1,4-butanediol diglycidyl ether as a crosslinking agent has a low crosslinking rate of 1%, when the gel reaches swelling equilibrium with a physiological saline, a storage modulus G' is about 1500Pa, and a loss modulus G" is about 300 to 400Pa in a range of a frequency of 0-1 to 10 Hz. From this, it was seen that, even when a different crosslinking agent from that of Example 1-1 is used, by performing a crosslinking reaction under the hyaluronic acid high concentration condition, a crosslinked hyaluronic acid gel having a low crosslinking rate and exhibiting excellent viscoelasticity is obtained.

### Example 1-3: 26W/V% hyaluronic acid. divinylsulfone crosslinking rate 0.8%

To 3000 µL of purified water were added 333 µL of a 2N NaOH aqueous solution and 2µL of divinylsulfone and, further, 1.0g of hyaluronic acid (Biohyalo 9: manufactured by Shiseido) was added. This mixture (storage modulus G' at frequency I Hz: 60000Pa) was stirred and mixed with a rotation/revolution mixer (AR250: manufactured by THINKY) at room temperature for 5 minutes. The product was allowed to stand in a physiological saline for one week until it reached swelling equilibrium. The swollen crosslinking hyaluronic acid gel was crushed with a sample mill (SK-M2: manufactured by KYORLTSU RIKO), to obtain a desired crosslinked hyaluronic acid gel slurry. The resulting crosslinked hyaluronic acid gel slurry was measured for a storage modulus G' and a loss modulus G" as in Example 1-1. Under presumption that a total amount of the added crosslinking agent reacted with a reactive group of hyaluronic acid, a crosslinking rate of the resulting crosslinked hyaluronic gel is 0.8% per one unit of a hyaluronic acid disaccharide repeating unit. The results of measurement are shown in Fig.6.

### Example 1-4: 26W/V% hyaluronic acid. 1,4-butanediol diglycidyl ether crosslinking rate 1%

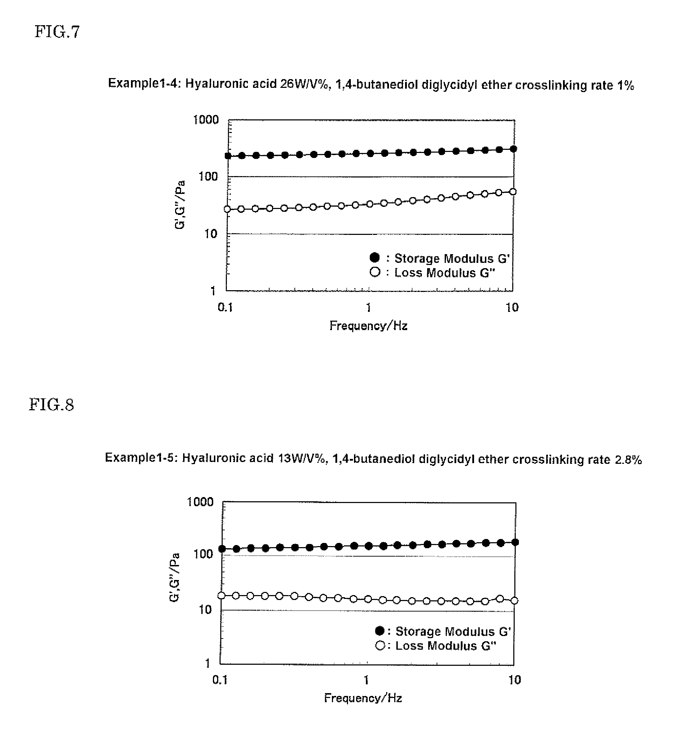

To 3000µL of purified water were added 333µL of a 2N NaOH aqueous solution, and 5µL of 1,4-butanediol diglycidyl ether and, further, 1.0g of hyaluronic acid (Biohyalo 12: manufactured by Shiseido) was added. This mixture (storage modulus G' at frequency 1Hz: 60000Pa) was stirred and mixed with a rotation/revolution mixer (AR-250: manufactured by THINKY) at room temperature for 5 minutes and this was allowed to stand at room temperature for 24 hours. The resulting gel was allowed to stand in a physiological saline for one week until the gel reached swelling equilibrium. The swollen crosslinking hyaluronic acid gel was crushed with a sample mill (SK-M2: manufactured by KYORITSU RIKO), to obtain a desired crosslinked hyaluronic acid gel slurry. The resulting crosslinked hyaluronic acid gel slurry was measured for a storage modulus G' and a loss modulus G" as in Example 1-1. Under presumption that a total amount of the added crosslinking agent reacted with a reactive group of hyaluronic acid, a crosslinking rate of the resulting crosslinked hyaluronic acid gel is around 1% per one unit of a hyaluronic acid disaccharide repeating unit. Results of measurement are shown in Fig.7.

### Example 1-5: 13W/V% hyaluronic acid, 1,4-butanediol diglycidyl ether crosslinking rate 2.8%

To 2250µL of purified water were added 250µL of a 2N NaOH aqueous solution, and 5µL of 1,4-butanediol glycidyl ether and, further, 0.375g of hyaluronic acid (Biohyalo 12: manufactured by Shiseido) was added. This mixture (storage modulus G' at frequency 1Hz: 20000Pa) was stirred and mixed with a rotation/revolution mixer (AR-250: manufactured by THINKY), and this was allowed to stand at room temperature for 24 hours. The resulting gel was allowed to stand in a physiological saline for one week until the gel reached swelling equilibrium. The swollen crosslinking hyaluronic acid gel was crushed with a sample mill (SK-M2: manufactured by KYORITSU RIKO), to obtain a desired crosslinked hyaluronic acid gel slurry. The resulting crosslinked hyaluronic acid gel slurry was measured for a storage modulus G' and a loss modulus G" as in Example 1-1. Under presumption that a total amount of the added crosslinking agent reacted with a reactive group of hyaluronic acid, a crosslinking rate of the resulting crosslinked hyaluronic acid gel is 2.8% per one unit of a hyaluronic acid disaccharide repeating unit_ The results of measurement are shown in Fig.8.

As shown in Fig.6, in the case of Example 1-3 using divinylsulfone as a crosslinking agent under the condition of a hyaluronic acid concentration of 26W/V%, a crosslinking rate was around 0.8% and, when the gel reached swelling equilibrium with a physiological saline, a storage modulus G' was about 300 to 700 Pa, and a loss modulus G" was about 100 to 200 Pa. In addition, as shown in Fig.7, in the case of Example 1-4 using 1,4-butanediol diglycidyl ether, a crosslinking rate was around 1% and, when the gel reached swelling equilibrium with a physiological saline, a storage modulus G' was about 200 Pa, and a loss modulus G" was about 30 to 50Pa. Further, as shown in Fig.8, in the crosslinked hyaluronic acid gel of Example 1-5 obtained under the condition of a hyaluronic acid concentration of 13W/V% using 1,4-butanediol diglycidyl ether as a crosslinking agent, a crosslinking rate was around 2.8% and, when the gel reached swelling equilibrium with a physiological saline, a storage modulus G' was about 150Pa, and a loss modulus G" was about 20Pa.
From these results, it was revealed that, by performing a crosslinking reaction under the hyaluronic acid high concentration condition, a crosslinked hyaluronic acid gel having excellent viscoelasticity can be obtained although a crosslinking rate is low such as around 0.8 to 2.8%.

### Study of hyaluronic acid concentration condition

Subsequently, in order to study the concentration condition of hyaluronic acid to be subjected to a crosslinking reaction in detail, the present inventors tried the same test as that of Example 1-1 by variously changing the hyaluronic acid concentration conditions, and studied relationship with viscoelasticity with a crosslinked hyaluronic acid gel.

### Examples 2-1. and 2.2. and Comparative Examples 2-1 to 2-5

To 4.7mL of purified water were added 0.25mL of a 2N NaOH aqueous solution, and 50µL of divinylsulfone which had been diluted 13.3-fold with dimethyl sulfoxide (3µL as divinylsulfone) and, further, various concentrations of hyaluronic acid (Biohyalo 12: manufactured by Shiseido) were added (Example 2-1: 1g (18.2W/V%), Example 2-2: 0.75g (13.9W/V%), Comparative Example 2-1: 0.5g (9.4W/V%), Comparative Example 2-2: 0.25g (5.0W/V%), Comparative Example 2-3: 0.15g (3.0W/V%), Comparative Example 2-4:0.1g (2.OW/V%), Comparative Example 2-5:0.05g (1.0W/V%)). This mixture was stirred and mixed with a rotation/revolution mixer (AR-250: manufactured by THINKY) at room temperature for 5 minutes. The product was allowed to stand in a physiological saline for one week until this gel reached swelling equilibrium. The swollen crosslinking hyaluronic acid gel was crushed with a sample mill (SK-M2: manufactured by KYORITSU RIKO) to obtain a desired crosslinked hyaluronic acid gel slurry. Regarding the resulting crosslinked hyaluronic acid gel, a storage modulus G' was measured with a rheometer (Rheolyst AR1000-N: manufactured by TA Instruments) under the condition of 25°C and a frequency of I Hz. The results are shown in the following in Table 1.

**Table 1**

| | Hyaluronic acid concentration in a mixture (W/V%) | Storage modulus G' (frequency 1Hz) |
|---|---|---|
| Example 2-1 | 18.2 | 627.7 |
| Example 2-2 | 13.9 | 204.1 |
| Comparative Example 2-1 | 9.4 | 0.17 |
| Comparative Example 2-2 | 5.0 | Unmeasurable (partially crosslinked gel) |
| Comparative Example 2-3 | 3.0 | Unmeasurable (partially crosslinked gel) |
| Comparative Example 2-4 | 2.0 | Unmeasurable (extremely partially crosslinked gel) |
| Comparative Example 2-5 | 1.0 | Unmeasurable (extremely partially crosslinked gel) |

As shown in Table 1, in Examples 2-1 and 2-2 where a hyaluronic acid concentration in a mixture is 13.9 to 18.2%, a storage modulus G' when the gel reached swelling equilibrium with a physiological saline is 627.7Pa or 204.1Pa, and it was made clear that a crosslinked hyaluronic acid gel having excellent viscoelasticity is obtained. To the contrary, in Comparative Example 2-1 where a hyaluronic acid concentration in a mixture is 9.4W/V%, a storage modulus G' was 0.17Pa, and extremely low viscoelasticity was exhibited. Further, in Comparative Examples 2-2 to 2-5 where a hyaluronic acid concentration in a mixture is 5.0W/V% or lower, although formation of a crosslinked gel was seen partially, a majority was a high concentrated hyaluronic acid solution, and a gel exhibiting viscoelasticity was not obtained.

### Preparation of crosslinked hyaluronic acid gel with dough kneading machine

Subsequently, using a commercial available dough kneading machine, the present inventors tried a crosslinking reaction with highly concentrated hyaluronic acid and a small amount of a crosslinking agent, and viscoelasticity of the resulting crosslinked hyaluronic acid gel was measured.

### Example 3-1

To 190mL of purified water were added 2mL of divinylsulfone solution which had been diluted 16.7-fold with dimethyl sulfoxide (120µL as divinylsulfone), this was mildly stirred and, further, 28.0g of hyaluronic acid (Biohyalo 12: manufactured by Shiseido) was added (hyaluronic acid concentration 13.0W/V%). This mixture was stirred and mixed with a commercially available dough kneading machine (National Food Processor MK-K58: manufactured by Matsushita Electric Industrial Co., Ltd., equipped with a separately sold bread wing (AUFB4- 1 3 7)) at room temperature for 2 minutes. Then the mixture was added 10mL af a 2N NaOH aqueous solution, further stirred and mixed for 5 minutes. The product was sealed in a sealable polyethylene bag, and this was allowed to stand at room temperature for 18 hours to obtain 200g of a crosslinked hyaluronic acid gel. About 7.2g of this crosslinked hyaluronic acid gel was allowed to stand in a physiological saline for one week until the gel reached swelling equilibrium, to obtain a swollen gel. Further, this swollen gel was slurried by grinding with a mill, about 1mL of this was filled into a small-type injection syringe, and high pressure steam sterilization was performed. After this high pressure steam sterilization, a storage modulus G' was measured under the condition of 25°C and a frequency of 1Hz using a rheometer (Rheolyst AR1000-N: manufactured by TA Instruments), and it was found to be 577Pa.

### Preparation of crosslinked hyaluronic acid gel by hand kneading

Subsequently, the present inventors tried a crosslinking reaction with highly concentrated hyaluronic acid and a small amount of a crosslinking agent by stirring and mixing by kneading with human hands, and viscoelasticity of the resulting crosslinked hyaluronic acid gel was measured.

### Examples 4-1 and 4,-2, Comparative Example 4-1

To a polyethylene bag (length 100mm, width 70mm) which had been disinfected with ethanol in advance were added 4.95mL of a 0.1N aqueous NaOH solution, and 50µL of divinylsulfone which had been diluted 13.3-fold with dimethyl sulfoxide (3µL as divinylsulfone), this was stirred well and, further, various concentrations of hyaluronic acid (Biohyalo 12: manufactured by Shiseido) were added (Example 4-1: 0.7g (13W/V%), Example 4-2: 0.6g (11 W/V%), Comparative Example 4-1: 0.5g (9.4W/V%). After addition of hyaluronic acid, the bag was sealed quickly, and the mixture in the bag was mixed well with two fingers of both hands by kneading. After mixed for about 5 minutes, the bag was swung to collect the gel-like mixture on the bottom of the bag, and this was molded into a bar. The bag was allowed to stand in a clean bench for 4 hours, and the bag was broken to take out the product, to obtain a crosslinked hyaluronic acid gel. This crosslinked hyaluronic acid gel was allowed to stand in a physiological saline for 1 week until this reached swelling equilibrium, to obtain a swollen gel. Further, this swollen gel was ground with a mill to slurry it, about 1mL of this was filled into a small-type injection syringe, and high pressure steam sterilization was performed. After this high pressure steam sterilization, a storage modulus G' was measured under the condition of 25°C and a frequency of 1Hz using a rheometer (Rheolyst AR1000-N: manufactured by TA Instruments), and it was found to be 181Pa (Example 4-1), 61.5Pa (Example 4-2), or 10.5Pa (Comparative Example 4-1).

When slurries of the aforementioned crosslinked hyaluronic acid gels obtained in respective Examples were rubbed on a finger, in hyaluronic acid gel slurries prepared by stirring and mixing with a machine (rotation/revolution mixer or dough kneading machine), the presence of an extremely small amount of hard gel fine particles was confirmed, but in hyaluronic acid gel slurries prepared by stirring and mixing with human hand kneading, the presence of the hard fine particles was not confirmed. From this, it is thought that, in the process for preparing a crosslinked hyaluronic acid gel of the present invention, stirring and mixing by kneading with human hands is more suitable.

### Enzyme digestion resistance of crosslinked hyaluronic acid gel

Subsequently, in view of application of a crosslinked hyaluronic acid gel slurry which is used by injecting into a living body such as antiwrinkle injections, the present inventors studied enzyme digestion resistance of a crosslinked hyaluronic acid gel slurry obtained by the present invention.

### Enzyme digestion resistance test

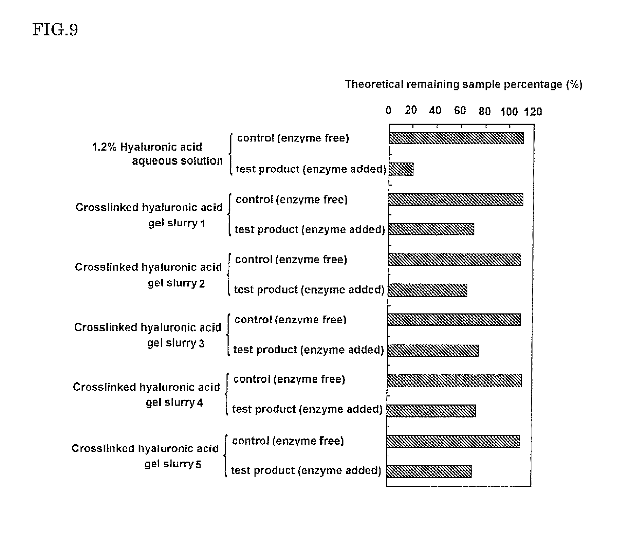

Each I g of crosslinked hyaluronic acid gel slurry 1 to 5 obtained by the process of the present invention, and a 1.2% hyaluronic acid aqueous solution was placed into a sterile Eppendorf tube (2mL), and each tube was placed into a centrifuge of a swing bucket, and centrifuged (8300 x g, 2000rpm, 10 minutes) in order to make a meniscus in the tube horizontal. Thereafter, 0.112mL of a hyaluronidase solution (bovine testis-extracted hyaluronidase, 2000 unit/mL, 0.1mol/mL, phosphate buffer pH 7.4) was gently overlaid on the hyaluronic acid surface in each tube, which was used as a test product. Separately, a sample obtained by the same manner except that hyaluronidase was not contained, served as the control. Each tube was placed into a constant temperature bath at 37°C, and an enzyme reaction was performed for 16 hours. The final enzyme activity of a test product after addition of an enzyme solution became 201 unit/mL. After the reaction, each tube was inverted, a liquidized sample was absorbed with a paper towel, and a weight of a sample remaining on a bottom of the tube was measured. Summary of results of measurement of a sample weight of test products and the control in respective samples, as a theoretical remaining sample percentage (%), is shown in Fig.9. The theoretical remaining sample percentage (%) is obtained by subtracting a weight of a liquefied sample from a theoretical initial sample weight (1g) to obtain a remaining sample weight, and expressing a remaining sample weight as percentage relative to a theoretical initial amount (1 g).

As shown in Fig.9, in the control in which an addition solution (0.112mL) not containing the enzyme was added, a weight of an added solution was increased, but liquefaction of a 1.2% hyaluronic acid aqueous solution or a crosslinked hyaluronic acid gel was not seen at all. On the other hand, it was seen that, among test products with the enzyme added thereto, in a 1.2% hyaluronic acid aqueous solution, about 80% was liquefied. To the contrary, in crosslinked hyaluronic acid gel slurry 1 to 5 obtained by the process of the present invention, liquefaction of a gel was only 35% to 25%. From this, it is thought that the crosslinked hyaluronic acid gel slurry obtained by the present invention has high enzyme digestion resistance (hyaluronidase resistance), and can maintain a gel structure also in a living body for a long term.

## Claims

1. A process for preparing a crosslinked hyaluronic acid gel, comprising stirring and mixing a mixture containing 10W/V% or more of hyaluronic acid, a crosslinking agent and water under acidic or alkaline condition.

2. The process for preparing a crosslinked hyaluronic acid gel according to claim 1, wherein a storage modulus G' (frequency 1Hz) of the mixture before subjected to a crosslinking reaction is 15000Pa or higher.

3. The process for preparing a crosslinked hyaluronic acid gel according to claim 1 or 2, wherein the mixture is stirred and mixed without physical cutting of a hyaluronic acid polymer chain in the mixture.

4. The process for preparing a crosslinked hyaluronic acid gel according to claim 3, wherein the mixture is stirred and mixed with a rotation/revolution mixer.

5. The process for preparing a crosslinked hyaluronic acid gel according to claim 3, wherein the mixture is stirred and mixed with a dough kneading machine or a rice-cake making machine.

6. The process for preparing a crosslinked hyaluronic acid gel according to claim 3, wherein the mixture is stirred and mixed by kneading with human hands.

7. The process for preparing a crosslinked hyaluronic acid gel according to any one of claims 1 to 6, wherein a crosslinking agent concentration in the mixture is 0.02 to 1W/V%.

8. The process for preparing a crosslinked hyaluronic acid gel according to any one of claims 1 to 6, wherein a crosslinking agent concentration in the mixture is 0.02 to 2W/W% relative to hyaluronic acid disaccharide repeating unit.

9. The process for preparing a crosslinked hyaluronic acid gel according to any one of claims I to 8, wherein the crosslinking agent is selected from the group consisting of divinylsulfone, 1,4-butanediol diglycidyl ether, and ethylene glycol diglycidyl ether.
